(19) Europäisches Patentamt
European Patent Office
Office européen des brevets

(11) Veröffentlichungsnummer: **0 005 233**
**B1**

(12) # EUROPÄISCHE PATENTSCHRIFT

(45) Veröffentlichungstag der Patentschrift: **11.07.84**

(51) Int. Cl.[3]: **C 07 C 119/055,** B 01 J 31/06, C 08 G 18/79, C 07 F 9/53

(21) Anmeldenummer: **79101243.8**

(22) Anmeldetag: **25.04.79**

(54) **Verfahren zur Herstellung von flüssigem, lagerstabilem, teilweise carbodiimidisiertem Methylen-bis-(phenylisocyanat), sowie seine Verwendung als Isocyanatkomponente bei der Durchführung des Isocyanat-Polyadditionsverfahrens.**

(30) Priorität: **05.05.78 US 903308**

(43) Veröffentlichungstag der Anmeldung: **14.11.79 Patentblatt 79/23**

(45) Bekanntmachung des Hinweises auf die Patenterteilung: **11.07.84 Patentblatt 84/28**

(84) Benannte Vertragsstaaten: **BE DE FR GB IT NL SE**

(56) Entgegenhaltungen:
**FR - A - 2 299 353**
**FR - A - 2 306 197**
**FR - A - 2 322 129**
**FR - A - 2 341 605**
**US - A - 3 152 162**

(73) Patentinhaber: **Mobay Chemical Corporation, Penn Lincoln Parkway West, Pittsburgh, Pennsylvania 15205 (US)**

(72) Erfinder: **Sundermann, Rudolf, Dr., Treptower-Strasse 5, D-5090 Leverkusen (DE)**
Erfinder: **Slack, William E., 2805 Glenwood Avenue, Moudsville, WV 26041 (US)**

(74) Vertreter: **Weber, Gerhard, Dr. et al, BAYER AG Zentralbereich Patente, Marken und Lizenzen, D-5090 Leverkusen, Bayerwerk (DE)**

**EP 0 005 233 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Methylen-bis-(phenylisocyanat) stellt eine gebräuchliche Polyisocyanat-Komponente bei der Herstellung von geschäumten und nicht-geschäumten Polyurethanen dar. Reines Methylen-bis-(phenylisocyanat), d.h. ein solches welches weniger als 5 Gew.-% an Polyisocyanaten einer über 2 liegenden Funktionalität aufweist, und welches im allgemeinen aus 4,4',-Diisocyanatodiphenylmethan und/oder 2,4'-Diisocyanatodiphenylmethan besteht, ist normalerweise bei Raumtemperatur fest. Das Material muss daher durch Erhitzen in den flüssigen Zustand überführt und während seiner Verarbeitung bei der erhöhten Temperatur gehalten werden. Es mangelte daher nicht an Versuchen, Methylen-bis-(phenylisocyanat) so zu modifizieren, dass es bei Raumtemperatur flüssig und lagerstabil ist.

Gemäss den US-Patenten 3 384 653 und 3 449 256 erhitzt man hierzu Methylen-bis-(phenylisocyanat) in Gegenwart eines Trialkylphosphats, so dass ein flüssiges, teilweise carbodiimidisiertes Polyisocyanatgemisch entsteht.

Gemäss US-Patent 3 152 162 erhitzt man Methylen-bis-(phenylisocyanat) in Abwesenheit von Katalysatoren unter teilweiser Carbodiimidisierung der Isocyanatgruppen. Das dabei erhaltene Produkt kristallisiert jedoch beim Lagern. Vermutlich ist dies auf Nebenreaktionen während des Erhitzens zurückzuführen.

Es wurde schliesslich auch vorgeschlagen, Methylen-bis-(phenylisocyanat) durch teilweise Carbodiimidisierung mittels Phospholinoxid-Katalysatoren und anschliessende Vernichtung des Katalysators durch Zugabe eines Katalysatorengifts zu verflüssigen. Gemäss US-Patent 4 014 935 erhitzt man hierzu das Ausgangsisocyanat auf eine unterhalb 150°C liegende Temperatur in Gegenwart von 1 bis 10 ppm eines Phospholinoxids. Die Umsetzung wird dann durch Zugabe eines Katalysatorengifts abgebrochen. Geeignete Katalysatorengifte sind Chlorwasserstoff, Phosphorchlorid, Zinnchlorid, Phosphoroxichlorid oder Schwefel. Gemäss einem anderen Vorschlag (US-Patent 4 088 665) wird das Isocyanat mit 0,1 bis 100 ppm eines Phospholinoxids bei 0 bis 200°C vermischt und die Umsetzung durch Zugabe eines Katlaysatorgifts abgebrochen.

Wie jetzt überraschend gefunden wurden, ist es auch möglich, bei Raumtemperatur flüssiges, lagerstabiles, teilweise carbodiimidisiertes Methylen-bis-(phenylisocyanat) unter Verwendung von Phospholinoxiden herzustellen, ohne den Katalysator durch Zugabe eines Katalysatorengiftes zu vernichten, wenn man den Katalyator in einer Menge von 0,05 bis 10 ppm (parts per million) eines Phospholinoxids bei 150 bis 300°C durchführt und anschliessend das Reaktionsgemisch auf 100°C oder eine tiefere Temperatur abschreckt.

Gegenstand der vorliegenden Erfindung ist ein Verfahren zur Herstellung von flüssigem, lagerstabilem, teilweise carbodimisiertem Methylen-bis-(phenylisocyanat) durch Erhitzen von Methylen-bis-(phenylisocyanat) in Gegenwart von Phospholinoxid, dadurch gekennzeichnet, dass man Methylen-bis-(phenylisocyanat) in Gegenwart von 0,05 bis 10 ppm eines Reaktionsgemisch nach Erreichen des gewünschten Isocyanatsgehalts auf 100°C oder eine niedrigere Temperatur abschreckt.

Gegenstand der vorliegenden Erfindung ist auch die Verwendung des nach diesem Verfahren erhaltenen Methylen-bis-(phenylisocyanats), als Isocyanatkomponente bei der Durchführung des Isocyanat-Polyadditonsverfahrens.

Für das erfindungsgemässe Verfahren geeignete Phospholinoxide sind beispielsweise die bekannten Verbindungen der allgemeinen Formeln.

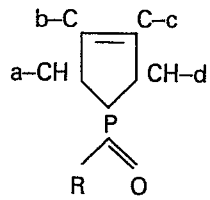

(I)

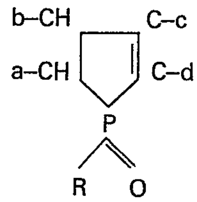

(II)

in welchen

R einen Alkylrest, vorzugsweise mit 1 bis 4 Kohlenstoffatomen, eine Phenylrest, einen Alkylrest, vorzugsweise mit 1 bis 4 Kohlenstoffatomen, Wasserstoff oder einen Alkenylrest, vorzugsweise mit 2 bis 4 Kohlenstoffatomen bedeutet,

a, b, c und d für gleiche oder verschiedene Reste stehen und jeweils Wasserstoff, ein Halogenatom, einen Alkylrest, vorzugsweise mit 1 bis 4 Kohlenstoffatomen, einen Alkenylrest, vorzugsweise mit 2 bis 4 Kohlenstoffatomen, einen Phenylrest oder einen Cyclohexylrest bedeuten, oder wobei zwei der Reste zusammen eine Polymethylen-Kette vorzugsweise mit 3 bis 5 Kohlenstoffatomen darstellen, die mit zwei benachbaren Kohlenstoffatomen des heterocyclischen Rings einen cycloaliphatischen Ring bilden.

Die für das erfindungsgemässe Verfahren geeigneten Phospholinoxide der genannten allgemeinen Formeln können nach bekannten Verfahren hergestellt werden (vgl. z. B. G. M. Kosolapoff, L. Maier, Organic Phosphorous Compounds, Wiley-Interscience, New York, 1972 et seq., 3, Seiten 370 bis 371, 458 bis 463 und 4, Seiten 9 bis 10 und 48).

Derartige Phospholinoxide werden auch in den US-Patenten 2 663 737m, 2 663 738m, 2 663 739m, 2 941 966 und 2 853 473 beschrieben. Typische Beispiele geeigneter Phospholinoxide sind 1-Methyl-1-oxophospholin, 1-Ethyl-1-oxo-phospholin, 1-Butyl-1-oxophospholin, 1-(2-Ethyl-hexyl)-1-oxophospholin, 1-(2-Chlor-ethyl)-1-oxophospholin, 1-Phenyl-1-oxophospholin, 1-p-Tolyl-1-oxophospholin, 1-Chlormethyl-1-oxophospholin, 1,3-Dimethyl-1-oxopohospholin, 1,2-Dimethyl-1-oxophospholin, 1-Methyl-3-chlor-1-oxophospholin, 1-Methyl-3-brom-1-oxophospholin, 1-Chlorphenyl-1-oxophospholin, 1,3,4-Trimethyl-1-oxophospholin, 1,2,4-Trimethyl-1-oxophospholin, 1,2,2-Trimethyl-1-oxophospholin, 1-Phenyl-3-methyl-1-oxophospholin und 1-Phenyl-2,3-dimethyl-1-oxophosphlin.

Diese Verbindungen weisen im allgemeinen entweder in 2,3- oder in 3,4-Stellung eine Doppelbindung auf. Im allgemeinen werden beim erfindungsgemässen Verfahren technische Isomerengemische eingesetzt, die sowohl 2,3- als auch 3,4-ungesättigte Verbindungen enthalten. Ganz besonders bevorzugt werden beim erfindungsgemässen Verfahren solche Verbindungen der o.g. Formeln eingesetzt, in welchem R einen unsubstituierten Alkylrest mit 1 bis 4 Kohlenstoffatomen, einen unsubstituierten Alkoxyrest mit 1 bis 4 Kohlenstoffatomen oder einen Phenylrest bedeutet und a, b, c und d gleiche oder verschiedene Reste darstellen und Wasserstoff oder einen unsubsituierten Alkylrest mit 1 bis 4 Kohlenstoffatomen bedeuten.

Bei dem beim erfindungsgemässen Verfahren einzusetzenden Methylen-bis-(phenylisocyanat) handelt es sich vorzugsweise um 4,4'-Diisocyanatodiphenylmethan oder aber auch um dessen Gemische mit bis zu 70, vorzugsweise bis zu 35 Gew.-%, bezogen auf Gesamtgemisch, an 2,4'-Diisocyanatodiphenylmethan, sowie gegebenenfalls, jedoch weniger bevorzugt, mit bis zu 5 Gew.-% an 2,2'-Diisocyanatodiphenylmethan. Der Begriff Methylen-bis-(phenylisocyanat) umfasst im Rahmen der vorliegenden Erfindung jedoch auch Abmischungen der genannten Isomeren bzw. Isomerengemische mit bis zu 30, vorzugsweise bis zu 15 Gew.-%, bezogen auf Gesamtgemisch, an höherfunktionellen Polyisocyanaten der Diphenylmethan-Reihe. Diese höherfunktionellen Polyisocyanate entstehen neben den genannten difunktionellen Diisocyanaten bei der Phosgenierung von Anilin/Formaldheyd-Kondensaten einer, den gemachten Mengenangaben entsprechenden Zusammensetzung. Diese Polyisocyanatgemische können beim erfindungsgemässen Verfahren ebenfalls eingesetzt werden. Die erfindungsgemässe Massnahme hat bei Verwendung dieser Gemische zur Folge, dass die Kristallisationsneigung der Polyisocyanatgemische bei Raumtemperatur herabgesetzt wird.

Zur Durchführung des erfindungsgemässen Verfahrens wird das teilweise zu carbodiimidisierende Ausgangsisocyanat in flüssigem bzw. aufgeschmolzenem Zustand, im allgemeinen bei 180 bis 300°C, vorzugsweise 180 bis 240°C und insbesondere 190 bis 210°C mit dem Katalysator vermischt. Die Carbodiimidisierungsreaktion wird dann bis zum gewünschten Carbodiimidisierungsgrad innerhalb der genannten Temperaturbereiche geführt und bei Erreichen des gewünschten Carbodiimidisierungsgrades durch Abschrecken, des Reaktionsgemisches auf 100°C oder eine niedrigere Temperatur abgebrochen. Oft ist es auch zweckmässig, den Katalysator dem Isocyanat vor Erreichen der obengenannten Reaktionstemperatur, d.h. bei ca. 20 bis 50°C zuzusetzen, vorausgesetzt, dass das Ausgangsdiidocyanat bei dieser Temperatur bereits flüssig bzw. geschmolzen vorliegt. Nach Zugabe des Katalysators erfolgt dann die Erhitzung des Gemischs auf die genannte Reaktionstemperatur.

Unter «Carbodiimidisierungsgrad» ist hierbei der Prozentsatz der Isocyanatgruppen des Ausgangsisocyanats zu verstehen, der während der erfindungsgemässen Umsetzung unter Kohlendioxid-Abspaltung in Carbodiimidgruppen überführt wird. Der Carbodiimidisierungsgrad kann während des Verfahrens entweder durch volumetrische Bestimmung des entwickelten Kohlendioxids oder durch Bestimmung des Isocyanatgehalts des Reaktionsgemischs festgestellt werden. Bei einem vorgewählten, mit dem erfindungsgemässen Verfahren anzustrebenden Carbodiimidisierungsgrad kann man daher vorab den Isocyanatgehalt des angestrebten Produkts errechnen und die Umsetzung nach Erreichen dieses gewünschten Isocyanatgehalts durch Abkühlen abbrechen. Beim erfindungsgemässen Verfahren wird die Carbodiimidisierungsreaktion im allgemeinen bei Erreichen eines Carbodiimidisierungsgrads von 5 bis 30%, abgebrochen.

Die Katalysatoren werden dabei in einer Menge von 1 ppb bis 10 ppm, vorzugsweise von 0,05 bis 5 ppm, bezogen auf das zu carbodiimidisierende Diisocyanat, eingesetzt. Die Zugabe des Katalysators erfolgt zum flüssigen bzw. gelösten Ausgangsisocyanat. Das erfindungsgemässe Verfahren wird vorzugsweise lösungsmittelfrei durchgeführt. Nach Erreichen des gewünschten Carbodiimidisierungsgrades wird die Reaktion durch Abschrecken auf 100°C oder eine tiefere Temperatur, vorzugsweise 20 bis 60°C, abgebrochen. Wegen der extrem niedrigen Konzentration des Katalysators kann es vorkommen, dass sich der Katalysator während der erfindungsgemässen Umsetzung verflüchtigt. In solchen Fällen kann es sich als notwendig herausstellen, eine weitere Menge an Katalysator während der Umsetzung zuzusetzen, wobei jedoch die Gesamtmenge des zugesetzten Katalysators 10 ppm nicht übersteigen sollte. Falls beim erfindungsgmässen Verfahren mehr als 10 ppm an Katalysator eingestzt werden, kann es vorkommen, dass das Endprodukt sich als bei Raumtemperatur nicht lagerstabil herausstellt.

Das erfindungsgemässe Verfahren kann diskontinuierlich oder kontinuierlich durchgeführt werden. Vorzugsweise erfolgt die Durchführung kontinuierlich. Bei dieser kontinuierlichen Arbeitsweise passiert beispielsweise das teilweise zu carbodiimidisierende Ausgangsisocyanat einen geeigneten Reaktor, beispielsweise ein beheiztes Reaktionsrohr oder einen beheizten Reaktionsturm, wobei der Katalysator dem Ausgangspolyisocyanat kontinuierlich zudosiert wird, bevor dieses die heisse Reaktionszone erreicht. Die Zugabe des Katalysators kann jedoch auch in der heissen Reaktionszone selbst erfolgen. Der Carbodiimidisierungsgrad kann bei einer solchen kontinuierlichen Arbeitsweise durch die Fliessgeschwindigkeit, durch die Reaktionszone, d.h. durch die Verweilzeit in der Reaktionszone, die Katalysatorenmenge

und die Reaktionstemperatur beeinflusst werden.

Sofort nach Verlassen der Reaktionszone wird das Reaktionsgemisch auf eine bei 100°C oder tiefer, vorzugsweise zwischen 20 und 60°C liegende Temperatur, abgeschreckt. Bei kontinuierlicher Arbeitsweise kann dieses Abschrecken beispielsweise mittels eines Wärmeaustauschers erfolgen. Das plötzliche Abkühlen hat die sofortige Beendigung der Carbodiimidisierungsreaktion zur Folge, wobei der kritische Temperaturbereich, in welchem Methylen-bis-(phenylisocyanat) zur Dimerisierungsreaktion neigt, übersprungen werden kann, so dass unerwünschte Nebenreaktionen, insbesondere eine unerwünschte Uretdionbildung, nicht stattfinden. Bekanntlich lagern sich Isocyanatgruppen bei Raumtemperatur an Carbodiimidgruppen unter Bildung von Uretonimin-Gruppen an, so dass es sich bei den erfindungsgemässen Verfahrensprodukten im wesentlichen um Uretonimin-Einheiten aufweisende Methylen-bis-(phenylisocyanate) handelt.

Ein besonderer Vorzug des erfindungsgemässen Verfahrens liegt in der Tatsache begründet, dass es die Herstellung von nur teilweise carbodiimidisierten Isocyanaten auf besonders einfache Weise gestattet, wobei die Verfahrensprodukte sich durch einen besonders geringen Gehalt an Fremdsubstanzen, beispielsweise an desaktivierten Katalysatoren, auszeichnen.

Die Isocyanatgruppen aufweisenden Carbodiimide (bzw. Uretonomine), die beim erfindungsgemässen Verfahren als Verfahrensprodukte erhalten werden bzw. ihre Lösungen in Carbodiimid- bzw. Uretonimin-freien Diisocyanaten stellen wertvolle Ausgansgamterialien für das Polyadditonsverfahren dar. Die erfindungsgemässe Verfahrensprodukte eignen sich zur Herstellung von weichen oder harten, geschäumten oder nichtgeschäumten Kunststoffen, wie sie ihrerseits beispielsweise zur Herstellung von Lacken, Beschichtungen, Filmen oder Formkörpern der unterschiedlichsten Art zum Einsatz gelangen. Unter Verwendung der erfindungsgemässen Verfahrensprodukte hergestellte Polyurethane enthalten Carbodiimid- oder Uretonimingruppen, die ihrerseits eine Stabilisierung von gegebenenfalls gleichzeitig vorliegenden Estergruppen gegenüber Hydrolyse und ausserdem eine Verminderung der Entflammbarkeit des Kunststoffs bewirken.

Die nachstehenden Beispiele dienen zur weiteren Erläuterung der vorliegenden Erfindung. Alle Mengenangaben beziehen sich auf Gew.-Teile bzw. Gew.-%. In den Beispielen wird als Phospholinoxid ein Isomerengemisch des 1-Methyl-1-oxo-phospholins (P-methyl-phospholinoxid) verwendet.

Beispiel 1

997 Teile 4,4'-Diisocyanatodiphenylmethan werden in einem 1 l Dreihalskolben auf 200°C erhitzt und mit 0,0173 Teilen einer 3%igen Phospholinoxidlösung in Toluol (0,5 ppm) vermischt. Nach 42 Minuten bei 200°C ist der NCO-Gehalt des Diisocyanats auf 30,45% abgefallen. Ein weiteres Erhitzen während 10 Minuten auf 200°C führte zu keiner weiteren Änderung des Isocyanatgehalts. Das Reaktionsgemisch wird auf 50°C abgeschreckt. Nach 5-tägigem Lagern bei 25°C war das Reaktionsprodukt noch flüssig und wies eine NCO-Gehalt von 29,2% auf.

Beispiel 2

Beispiel 1 wurde unter Verwendung von 1506 Teilen 4,4'-Diisocyanataodiphenylmethan, 0,0556 Teilen der 3%igen Phospholinoxid-Lösung (1 ppm) bei einer Reaktionstemperatur von 198°C und einer Reaktionszeit von 40 Minuten wiederholt. Das Reaktionsgemisch wird auf 50°C abgeschreckt. Die entstehende Flüssigkeit wies einen Gehalt an Isocyanatgruppen von 26,6% auf. Nach 5-tägigem Lagern bei 25°C betrug der NCO-Gehalt der Flüssigkeit 25,9%.

Beispiel 3

Unter Verwendung der gleichen Apparatur wie in Beispiel 1 wurde bie 50°C 1 ppm Phospholinoxid zu 4,4'-Diisocyanatodiphenylmethan gegeben. Während eines Zeitraum von 30 Minuten wurde das Reaktionsgemisch auf 200°C erhitzt und bei dieser Temperatur während 14 Minuten gehalten. Nach Abschrecken auf 50°C und Lagerung bei Raumtemperatur über Nacht wies das Produkt einen NCO-Gehalt von 26,5% auf. Nach 5-tägigem Lagern bei 25°C lag eine lagerstabile Flüssigkeit mit einem NCO-Gehalt von 26,1% vor.

Beispiel 4

118 kg 4,4'-Diisocyanatodiphenylmethan werden in einem 189 l fassenden Reaktor aus nichtrostendem Stahl auf 197°C erhitzt. Bei dieser Temperatur wurden 0,8 g einer 3%igen Phospholinoxidlösung in Toluol (0,2 ppm) hinzugegeben. Nach 43 Minuten wurden weitere 0,8 g der Katalysatorlösung hinzugefügt. Nach weiteren 75 Minuten wurde das Reaktionsgemisch auf 80°C abgeschreckt. Es entstand ein flüssiges Reaktionsprodukt mit einem NCO-Gehalt von 29,8%.

Beispiel 5

Beispiel 1 wurde unter Verwendung von 482 Teilen 4,4'-Diisocyanatodiphenylmethan, 0,0085 Teilen der 3%igen Phospholinoxidlösung (0,5 ppm) bei einer Reaktionstemperatur von 180°C und einer Reaktionszeit von 60 Minuten wiederholt. Das Reaktionsgemisch wurde auf 50°C abgeschreckt. Die enstehende Flüssigkeit wies einen NCO-Gehalt von 28,9% auf. Nach 5-tägigem Lagern bei 25°C stellte sich der NCO-Gehalt der Flüssigkeit auf 27,1% ein.

Beispiel 6

Beispiel 1 wurde unter Verwendung von 481 Teilen 4,4'-Diisocyanatodiphenylmethan, 0,0088 Teilen der 3%igen Phospholinoxidlösung (0,5 ppm) bei einer Reaktionstemperatur von 190°C und einer Reaktionszeit von 57 Minuten wiederholt. Das Reaktionsgemisch wurde auf 50°C abgeschreckt. Die entstehende Flüssigkeit wies einen NCO-Gehalt von 28,7% auf. Nach 5-

tägiger Lagerung bei 25°C stellte sich der NCO-Gehalt der Flüssigkeit auf 26,8 % ein.

Beispiel 7

Beispiel 1 wurde unter Verwendung von 505 Teilen 4,4'-Diisocyanatodiphenylmetan, 0,009 Teilen der 3%igen Phospholinoxidlösung bei einer Reaktionstemperatur von 200°C und einer Reaktionszeit von 56 Minuten wiederholt. Das Reaktionsgemisch wurde auf 50°C abgeschreckt. Die entstehende Flüssigkeit wies einen NCO-Gehalt von 28,4% auf. Nach 5-tägigem Lagern bei 25°C stellte sich der NCO-Gehalt der Flüssigkeit auf 26,5% ein.

Beispiel 8

Beispiel 1 wurde unter Verwendung von 501 Teilen 4,4'-Diisocyanatodiphenylmethan, 0,0084 Teilen der 3% Phospholinoxidlösung (0,5 ppm) bei Reaktionstemperatur von 210°C und einer Reasktionszeit von 54 Minuten wiederholt. Das Reaktionsgemisch wurde auf 50°C abgeschreckt. Die entstehende Flüssigkeit wies einen NCO-Gehalt von 28,1% auf. Nach 5-tägigem Lagern bei 25°C stellte sich der NCO-Gehalt der Flüssigkeit auf 26,5% ein.

Beispiel 9

Beispiel 1 wurde unter Verwendung von 420 Teilen 4,4'-Diisocyanatodiphenylmethan, 0,0135 Teilen der 3%igen Phospholinoxidlösung (1 ppm) bei einer Reaktionstemperatur von 180°C und einer Reaktionszeit von 71 Minuten wiederholt. Das Reaktionsgemisch wurde auf 50°C abgeschreckt. Die entstandene Flüssigkeit wies einen NCO-Gehalt von 23,3% auf. Nach 5-tägigem Lagern bei 25°C stellte sich der NCO-Gehalt der Flüssigkeit auf 20,5% ein.

Beispiel 10

Beispiel 1 wurde unter Verwendung von 452 Teilen 4,4'-Diisocyanatodiphenylmethan, 0,016 Teilen der 3%igen Phospholinoxidlösung (1,0 ppm) bei einer Reaktionstemperatur von 190°C und einer Reaktionszeit von 62 Minuten wiederholt. Das Reaktionsgemisch wurde auf 50°C abgeschreckt. Die entstandene Flüssigkeit wies einen NCO-Gehalt von 23,2% auf. Nach 5-tägigem Lagern bei 25°C stellte sich der NCO-Gehalt der Flüssigkeit auf 20,3% ein.

Beispiel 11

Beispiel 1 wurde unter Verwendung von 547 Teilen 4,4'-Diisocyanatodiphenylmethan und 0,0188 Teilen der 3%igen Phospholinoxidlösung (1,0 ppm) bei einer Reaktionstemperatur von 200°C und einer Reaktionszeit von 32 Minuten wiederholt. Das Reaktionsgemisch wurde auf 25°C abgeschreckt. Die entstandene Flüssigkeit wies einen NCO-Gehalt von 23,3% auf. Nach tägigem Lagern bei 25°C stellte sich der NCO-Gehalt der Flüssigkeit auf 20,5% ein.

Beispiel 12

Beispiel 1 wurde unter Verwendung von 498 Teilen 4,4'-Diisocyanatodiphenylmethan und 0,0166 Teilen der 3%igen Katalysatorlösung (1 ppm) bei einer Reaktionstemperatur von 210°C und einer Reaktionszeit von 29 Minuten wiederholt. Das Reaktionsgemisch wurde auf 50°C abgeschreckt. Die entstandene Flüssigkeit wies einen NCO-Gehalt von 23,1% auf. Nach 5-tägigem Lagern bei 25°C stellte sich der NCO-Gehalt der Flüssigkeit auf 20,1% ein.

Beispiel 13

Beispiel 1 wurde unter Verwendung von 493 Teilen 4,4'-Diisocyanatodiphenylmethan und 0,032 Teilen der 3%igen Phospholinoxidlösung (2,0 ppm) bei einer Reaktionstemperatur von 180°C und einer Reaktionszeit von 20 Minuten wiederholt. Das Reaktionsgemisch wurde auf 50°C abgeschreckt. Die entstandene Flüssigkeit wies einen NCO-Gehalt von 23,4% auf. Nach 5-tägigem Lagern bei 25°C stellte sich der NCO-Gehalt der Flüssigkeit auf 20,7% ein.

Beispiel 14

Beispiel 1 wurde unter Verwendung von 469 Teilen 4,4'-Diisocyanatodiphenylmethan und 0,0320 Teilen der 3%igen Phospholinoxidlösung (2,0 ppm) bei einer Reaktionstemperatur von 190°C und einer Reaktionszeit von 19 Minuten wiederholt. Das Reaktionsgemisch wurde auf 50°C abgeschreckt. Die entstandene Flüssigkeit wies einen NCO-Gehalt von 23,3% auf. Nach 5-tägigem Lagern bei 25°C stellte sich der NCO-Gehalt der Flüssigkeit auf 20,5% ein.

Beispiel 15

Beispeil 1 wurde unter Verwendung von 502 Teilen 4,4'-Diisocyanatodiphenylmethan und 0,0390 Teilen der 3%igen Phospholinoxidlösung bei einer Reaktionstemperatur von 200°C und einer Reaktionszeit von 17 Minuten wiederholt. Das Reaktionsgemisch wurde auf 50°C abgeschreckt. Die entstandene Flüssigkeit wies einen NCO-Gehalt von 23,3% auf. Nach 5-tägigem Lagern bei 25°C wies die Flüssigkeit einen NCO-Gehalt von 20,5% auf.

Beispiel 16

Beispiel 1 wurde unter Verwendung von 470 Teilen 4,4'-Diisocyanatodiphenylmethan und 0,0313 Teilen der 3%igen Phospholinoxidlösung (2,0 ppm) bei einer Reaktionstemperatur von 210°C und einer Reaktionszeit von 15 Minuten wiederholt. Das Reaktionsgemisch wurde auf 50°C abgeschteckt. Die entstandene Flüssigkeit wies einen NCO-Gehalt von 23,3% auf. Nach 5-tägigem Lagern bei 25°C stellte sich der NCO-Gehalt der Flüssigkeit auf 20,5% ein.

Beispiel 17

159 kg 4,4'-Diisocyanatodiphenylmethan wurden in einem 189 l fassenden Reaktor aus nichtrostendem Stahl auf 194°C erhitzt. Bei dieser Temperatur wurden 1,58 g der 3%igen Phospholinoxidlösung in Toluol (0,3 ppm) hinzugefügt. Nach 35 Minuten wurden weitere 1,58 g der Katalysatorlösung hinzugegeben. Nach weiteren 84 Minuren wurde das Reaktionsgemisch auf 80°C abgeschreckt, wonach ein flüssiges Reaktionsgemisch mit einem NCO-Gehalt von 26,3% vorlag.

Beispiel 18

Ein kontinuierlicher Versuch wurde durchgeführt, indem 4,4'-Diisocyanatodiphenylmethan, welches 1,0 ppm (Gew.-Teile) Phospholinoxid enthielt, so durch einen auf 200°C erhitzten Reaktor gepumpt wurde, dass eine Verweilzeit von 2 Stunden im Reaktor resultierte. Das den Reaktor verlassende Produkt wies einen NCO-Gehalt von 27,5% auf.

Beispiel 19

Beispiel 18 wurde mit dem einzigen Unterschied wiederholt, dass das 4,4'-Diisocyanatodiphenylmethan lediglich 0,5 ppm Phospholinoxid enthielt. Es resultierte ein Produkt mit einem NCO-Gehalt von 30,8%.

## Patentansprüche

1. Verfahren zur Herstellung von flüssigem, lagerstabilem, teilweise carbodiimidisiertem Methylen-bis-(phenylisocyanat) durch Erhitzen von Methylen-bis-(phenylisocyanat) in Gegenwart von Phospholinoxid, dadurch gekennzeichnet, dass man Methylen-bis-(phenylisocyanat) in Gegenwart von 0,05 bis 10 ppm eines Phospholinoxids auf 180°C bis 300°C erhitzt und das Reaktionsgemisch nach Erreichen des gewünschten Isocyanatsgehalts auf 100°C oder eine niedrigere Temperatur abschreckt.

2. Verfahren gemäss Anspruch 1, dadurch gekennzeichnet, dass man als Phospholinoxid ein p-Methyl-phospholinoxid-Isomerengemisch verwendet.

3. Verfahren gemäss Anspruch 1 und 2, dadurch gekennzeichnet, dass man die Umsetzung im Temperaturbereich von 180°C bis 240°C durchführt.

4. Verfahren gemäss Anspruch 1 bis 3, dadurch gekennzeichnet, dass man die Umsetzung im Temperaturbereich von 190 bis 210°C durchführt.

5. Verfahren gemäss Anspruch 1 bis 4, dadurch gekennzeichnet, dass man die Umsetzung durch Abschrecken auf 20 bis 60°C abbricht.

6. Verwendung des gemäss Anspruch 1 bis 5 erhaltenen Methylenen-bis-(phenylosocyanats) als Isocyanatkomponente bei der Durchführung des Isocyanat-Ployadditonsverfahrens.

## Claims

1. Process for the preparation of liquid, storage-stable, partially carboiimidised methylene-bis-(phenylisocyanate) by heating methylene-bis-(phenylisocyanate) in the presence of phospholine oxide, characterised in that methylene-bis-(phenylisocyanate) is heated to from 180°C to 300°C in the presence of 0.05 to 10 ppm of a phospholine oxide and the reaction mixture is cooled rapidly to 100°C or a lower temperature after the desired isocyanate content has been reached.

2. Process according to Claim 1, characterised in that an isomeric mixture of p-methyl-phospholine oxide is used as the phospholine oxide.

3. Process according to Clasims 1 and 2, characterised in that the reaction is carried out in the temperature range of from 180°C to 240°C.

4. Process according to Claim 1 to 3, characterised in that the reaction is carried out in the temperature range of from 190 to 210°C.

5. Process according to Claim 1 to 4, characterised in that the reaction is terminated by cooling rapidly to from 20 to 60°C.

6. Use of the methylenene-bis-(phenylisocyanate) obtained according to Claim 1 to 5 as the isocyanate component for carrying out the isocyanate polyaddition process.

## Revendications

1. Procédé de production de méthylène-bis-(phénylisocyanate) partiellement carbodiimidé liquide, stable à l'entreposage, par chauffage de méthylène-bis-(phénylisocyanate) en présence d'oxyde de phospholine, caractérisé en ce qu'on chauffe du méthylène-bis-(phénylisocyanate) en présence de 0,05 à 10 ppm d'un oxyde de phospholine à 180 – 300°C et on refroidit brusquement le mélange réactionnel à 100°C ou à une température plus basse après que la teneur en isocyanate désirée a été atteinte.

2. Procédé suivant la revendication 1, caractérisé en ce qu'on utilise comme oxyde de phospholine un mélange d'isomères d'oxyde de p-méthyl-phospholine.

3. Procédé suivant les revendications 1 et 2, caractérisé en ce qu'on conduit la réaction dans l'intervalle de températures de 180 à 240°C.

4. Procédé suivant les revendivcations 1 à 3, caractérisé en ce qu'on conduit la réaction dans l'intervalle de température de 190 à 210°C.

5. Procédé suivant les revendications 1 à 4, caractérisé en ce qu'on arrête la réaction par refroidissement brusque à 20 – 60 °C.

6. Utilisation du méthylène-bis-(phénylisocyanate) obtenu suivant les revendications 1 à 5, comme composant isocyanate dans la mise en oeuvre du procédé de polyaddition d'isocyanate.